# EUROPEAN PATENT APPLICATION

(11) **EP 4 528 694 A1**
(43) Date of publication of application: **26.03.2025**
(21) Application number: 23213119.3
(22) Date of filing: 29.11.2023
(51) Int. Cl.: G09B 19/00, A63B 24/00, G16H 20/30

(54) **METHOD AND SYSTEM TO IMPROVE PHYSICAL TRAINING IN CIRCUIT**

(30) Priority: 23.09.2023 BR 102023019509
(71) Applicant: 40 Minutes Empreendimentos Ltda, 04511-001 São Paulo (BR)
(72) Inventor: SECALI, Luiz Fernando, 04511-001 São Paulo (BR)
(74) Representative: Mola, Edoardo

(57) **Abstract**

The present invention relates to a physical training system, particularly circuit training, and a method for implementing an electronic personal-guided physical training system.

The training method and system of group physical exercises in the form of circuit comprise for each user the following steps:
(a) choose through an application on the mobile or computer or tablet, the day, time, the exercise program of the day (which will indicate which will be the starting station of the exercise) and the position at the indicated starting station, provided that it is available and has not been pre-scheduled by another user;
(b) guide through a screen with an electronic personal trainer, in each of the 3 to 16 stations each of the users, where on this screen it has the identification of the user, in order to perform at least 3 types of exercises, totaling the time of 5 to 20 minutes in each station, with at least 3 sets of repetitions for each exercise per station; totaling an exercise program of 30 to 80 minutes, two different exercise programs can be performed per day, preferably every 10 minutes starting a new exercise program, and occurring simultaneously in alternating or subsequent stations according to the periodization of each exercise plan carried out by the technical staff;
(c) wait for the time of user transitions between stations, which may vary from 10s to 90s;
(d) start the training at the initial station that was indicated, according to the exercise program chosen to perform on the day, as identified by the user on the screens;
(e) complete the training at the last station indicated.

## Description

### FIELD OF APPLICATION

The present invention relates to a method and system of physical training, particularly of circuit training.

### BACKGROUND

With medical research indicating the beneficial aspects of exercise in light of the decreases in physical activity resulting from modern lifestyles, there is a growing need for exercise programs that achieve beneficial physical activity in a method that is compatible with people's daily lives.

Fitness centers are already known and are based on the availability of a set of exercise equipment in a fitness or physical training room. In general, the user chooses the devices randomly, or in a specific order prescribed by an instructor, or in an order dictated by the availability of such equipment. A first disadvantage of this situation is the increase in the time required to complete an exercise program, since the sequence of exercise set depends on the aforementioned availability. Another obstacle lies in the fact that the user does not always receive feedback on their performance, resulting in the incorrect execution of some exercises, making them less efficient or even harmful.

Document US 2006223674 teaches a system and method for efficient automation of a fitness circuit. The system includes several networked client computers positioned at exercise stations. The client computers are coordinated by a primary client computer that serves as a relay between the client computers and a centralized circuit control system where circuit programming and exercise details are defined. The method includes allowing a practitioner to scan an ID card issued at each exercise station. A client computer positioned at the exercise station retrieves and displays specific exercise information for the user and further instructs the user when to advance to the next circuit station.

Document US 2009263772 teaches a method for teaching group physical activity. The method includes: selecting a video, wherein the video displays instructions for at least one physical activity; assembling selected exercise equipment, wherein the exercise equipment is selected based on the instructions displayed in the video and the number of participants in the group; positioning the exercise equipment in selected positions, wherein the exercise equipment is positioned based on the instructions displayed in the video; assigning participants to stations, wherein participants are assigned to stations based on the instructions displayed in the video; playing the video, wherein participants are instructed to participate in at least one physical activity following the instructions displayed in the video; and monitoring compliance, technique, and condition of participants as participants perform aspects of at least one physical activity.

Document BR112019013028A2 teaches a modular and integrated system for optimal, organized, structured and integral training and competition with the use of conditioning and training tools based on boxing and martial arts, record keeping, integration, display components and communication. The only structural elements of the invention comprise a metal frame, easy to assemble and disassemble, anchored to the floor with engaging means. Impact receiving means, boxing training bags, and other training tools may be mounted on the frame in a predetermined pre-structured arrangement and layout.

Document US2012309551A1 relates to a structured space for physical exercise practice and a method of managing physical exercise practice that includes a plurality of successive modules having at least one of the modules at least two exercise positions as well as a time counting means common to all modules. The count includes a plurality of periods of equal duration, each period being subdivided into (n) phases, these phases including a plurality (n-1) of exercise performance phases and a recovery and instruction phase. There are videos positioned in the direction of each module recording training films, and an employee program is able to associate each person in training by image.

Both strength and cardiovascular training are considered important aspects of an effective physical fitness program. Proven benefits of physical exercise include, for example, increased muscle mass, reduced resting heart rate, decreased cholesterol levels, reduced blood pressure, and less stress on joints. To achieve these benefits, a consistent exercise program that spans a long period of time is usually required. Common barriers to maintaining a consistent exercise program include, for example, time constraints, boredom, planning and follow-up overheads, lack of resistance, and budget limitations. Circuit training has become a common method of training that can minimize these barriers, and the present invention aims to optimize it to facilitate the greatest possible adherence of people.

### OBJECTIVES OF INVENTION

In this sense, the goal was to develop a physical training method and system based on the organization of training time, with flexibility of exercise schedule, as preferably every 10 minutes a new 40-minute exercise program begins, the system being electronically managed by an electronic personal trainer and also one or more physical educator(s) present in the physical training space.

In addition to schedule flexibility, the user can utilize multiple exercise modalities in a single exercise program.

Each person can improve their own performance according to their physical condition and goal, guided by the technical staff.

The fact that the sets are governed by time counting allows each user to dictate their number of repetitions and the load to be used according to their goal, fitness and pace, and can be guided by the technical staff.

The exercises may or may not be divided into types of exercises by muscle groups on weekdays, or according to the training periodization established by the technical staff.

### BRIEF DESCRIPTION OF THE FIGURES

The accompanying Figures illustrate the scope of the invention as follows:
Figure 1: presents a training site comprising 8 stations that present 3 types of exercises in each station;
Figure 2: presents a training site comprising 8 stations that present 5 types of exercises in each station.

### SUMMARY DESCRIPTION

The present invention refers to a method and system for training group physical exercises in the form of a circuit that comprises for each user:
(a) to choose through a mobile or computer or tablet application, the day, time, exercise program of the day (which will indicate which will be the starting station of the exercise) and the position at the indicated starting station, provided that it is available and has not been pre-scheduled by another user;
(b) to guide through a screen with an electronic personal trainer, in each of the 3 to 16 stations each of the users, where on this screen it has the identification of user, in order to perform at least 3 types of exercises, totaling the time of 5 to 20 minutes in each station, with at least 3 sets of repetitions for each exercise per station; totaling an exercise program of 30 to 80 minutes, two different exercise programs can be performed per day, starting a new exercise program every 5 to 20 minutes, and occurring simultaneously in alternating or subsequent stations, which are defined according to the periodization of each exercise program carried out by the technical staff;
(c) to wait for the time of user transitions between stations, which may vary from 10s to 90s;
(d) to start the training at the initial station that was indicated according to the exercise program chosen to perform on the day, as identified by the user on the screens;
(e) to complete the training at the last station indicated.

### DETAILED DESCRIPTION

The present invention describes a training method and system of the present invention where each user can perform their exercise by scheduling day, time and choosing their exercise program of the day (which will indicate which will be the starting station of the exercise) and the position in the indicated starting station, through an application or advice at the place's reception, in a place comprising from 3 to 16 exercise stations, preferably from 4 to 8 exercise stations, whose exercise block can vary from 5 to 20 min in each of the stations, scheduled to offer at least 3 different types of exercises in each station, with at least 3 sets of repetitions of each exercise, transition period from one exercise to the other and a transition time from one station to the other, and the instructions for one of the individuals in particular are indicated through a program on a screen in each station, where they can be indicated: which exercise to be done by each user, the count of the time of each exercise, identification of the user (name or CPF [Individual Taxpayer Registration] or nickname of the user), heart rate of the user, target zone and caloric expenditure, count of transition from exercise to exercise and the count of transition time from station to station.

The identification of the user on the screens can be given through their name, or nickname, or official document number such as the CPF.

The transition time from exercise to exercise within a same station may range from 10s to 60s, and the transition time from one station to another station may range from 10s to 90s.

The present invention deals with a method for group physical exercise training where each individual can perform their exercise by scheduling day, time and choosing an exercise program (which will indicate which will be the starting station of the exercise) and the position at the indicated starting station, provided that it is available and has not been pre-scheduled by another user, through an application (commanded by a software), or through the advice at the place's reception.

The training method and system according to the invention employs software that:
- allows for the guidance of the exercises through an electronic personal trainer and the rotation of the exercise, being able to start in a new station a new exercise block every 5 to 20 minutes; and a new exercise program every 5 to 20 minutes;
- makes it possible for two different exercise programs to occur simultaneously, using different stations.
- controls: the interval time between one set and another, the transition time between one exercise and another, the transition time between one station and another;
- allows for the visualization on the screens of the correct way of performing the exercises, through the electronic personal trainer.

The user registers in the application or via advice at the place's reception, where their personal data is collected within the software that generates the integration of all information with the training method program.

Class scheduling is done by an app available for IOS and Android that has class scheduling features or through the advice at the place's reception, and the user can choose the exercise program of the day and its position at the start station that was indicated, and a new user can enter every 10 minutes, which is equivalent to the predetermined time to perform the exercise block within each of the stations.

A proprietary software and application was developed to manage the training method. The software allows for the guidance of the exercises and the rotation of exercise, which is also a differential of the present invention, being able to start, preferably, a new exercise program, from 5 to 20 minutes, preferably every 10 minutes.

The exercise scheduling can be done through the app or through the advice at the place's reception.

The physical training method or system is based on a mix of stimuli from strength training, endurance, strength endurance, flexibility, weight training, cardiovascular, aerobic, anaerobic, fitness, physical therapy, balance, mobility, functional, HIIT, calisthenics, stretching, among others.

The exercises can be distributed by muscle group or through the periodization of classes defined by the technical staff. The method or system can employ up to 16 stations, preferably 8 stations being used 4 stations in each exercise program, thus having 2 exercise programs occurring simultaneously in the same location. The selection of exercises/stimuli and periodization of exercises, established by the technical staff, dictate the stations to be used that day in each exercise program. Containing numerous exercise variations and exercise stimuli.

The training site comprises 3 to 16 exercise stations.

In the stations there are weight training, aerobics, fighting, functional, cross training, cardio, Pilates, yoga, ginástica natural or natural gymnastics, dance, muay Thai, boxing, jiu-jitsu, spinning, running, air bike, flexibility, HIIT, among others. Dumbbell exercise material, dumbbell, air bike, step, jump, treadmill, naval rope, spinning bike, exercise bike, weighted ball, TRX, mat, kettlebell, elastic, paddle, balance platform, Bosu, sled, fighting bob, rope, tire, small round cone, small triangular cone, balance board, exercise ladder, plyometric box, ladder, mil ladder, among others.

An initial physical assessment or bioimpedance can be performed to guide the user's exercise according to their pace and goal. There is an evolution because there is an exercise guidance according to each person's physical condition and goals.

The exercises are distributed according to their periodization, determined by the technical staff.

Each station can indicate exercises whose exercise block in the station ranges from 5 to 20 min, each of the stations is scheduled to offer at least 3 different types of exercises, with at least 3 sets of repetitions of each exercise and transition period from 10s to 90s to move to the next station, and the instructions for one of the users in particular are indicated on a screen in each station.

In all stations there is a screen where it is indicated (by the avatar - the electronic personal trainer) in real time which exercise to be done by each user, how it must be performed in the correct way, the time count of each exercise, identification of the user (name or CPF or nickname of the user), whose characteristics and conditions are controlled by a program or software.

Each station has a screen where the demonstration of the 3 different types of exercises that will be performed in each station is made, where an avatar, the electronic personal trainer, indicates how the exercise should be correctly performed in real time.

Each user maintains their own pace according to their goal to be achieved, and controls the intensity and rest during the training set within each of the exercise stations. Each user dictates their own pace according to their physical conditions, limits and goals to be achieved.

According to the preferred way of performing the method according to the invention, it comprises two different types of 40-minute exercise program, which take place simultaneously, and each exercise program uses 4 exercise stations, totaling 8 available stations (the stations to be used in each exercise program of the day is defined by the technical staff and contemplates the types of exercises to be performed in each program), where 3 sets of 40 seconds of each exercise are performed with a transition period to rest of 20 seconds in each station. Preferably, a new exercise program is started every 10 minutes at each starting station.

According to another embodiment of the method according to the invention, it comprises a 30-minute exercise program distributed over 3 stations.

According to another embodiment of the method according to the invention, it comprises an exercise program of 40 minutes distributed over 4 stations.

According to another embodiment of the method according to the invention, it comprises a 50-minute exercise program distributed over 5 stations.

According to another embodiment of the method according to the invention, it comprises a 60-minute exercise program distributed over 6 exercise stations.

According to another embodiment of the method according to the invention, it comprises a 70-minute exercise program distributed over 7 exercise stations.

According to another embodiment of the method according to the invention, it comprises an 80-minute exercise program distributed over 8 or 16 exercise stations.

The training location can also include the personal trainer avatar, kids space, pet space, space with physiotherapist, individual sauna, cryotherapy, individual bathroom, yoga capsule, floating tub, personalized recovery space, parking for electric car recharging, cell phone recharging totem, amenities and towels service, stretching and mobility space.

One of the great advantages of the present invention is the extensive schedule during the day, in which a new exercise program is started every 10 minutes (16 hours of class per day), and may also include an area of recovery, bioimpedance, physical assessment, integrated classes with headphones, private label brand products focused on health, brand merchandise (t-shirts, caps, bottles, wind break, hoodie, sweatshirts, fitness clothes, etc).

According to one embodiment of the present invention, the training is carried out in a place that comprises two exercise programs in a total of 8 exercise stations, preferably 4 stations per exercise program are used simultaneously, according to the periodization of the exercises of that given day, determined by the technical staff.

The training site can present the distributed stations, side by side, of free transit for users, that is, there are no walls or structures that isolate each station, leaving free the transit of users between stations or being able to present the 4 stations on the ground floor of free transit for users, and 4 stations of free transit on the mezzanine or first floor.

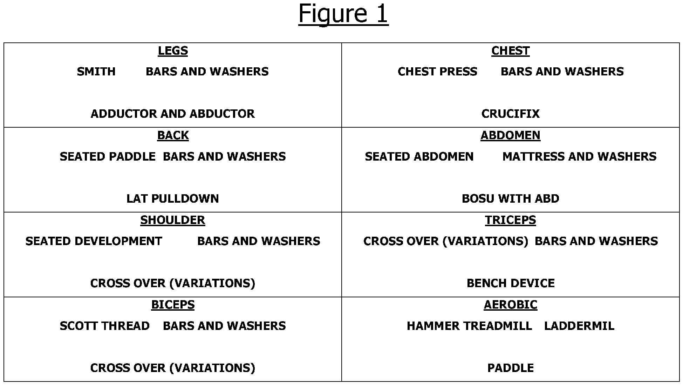

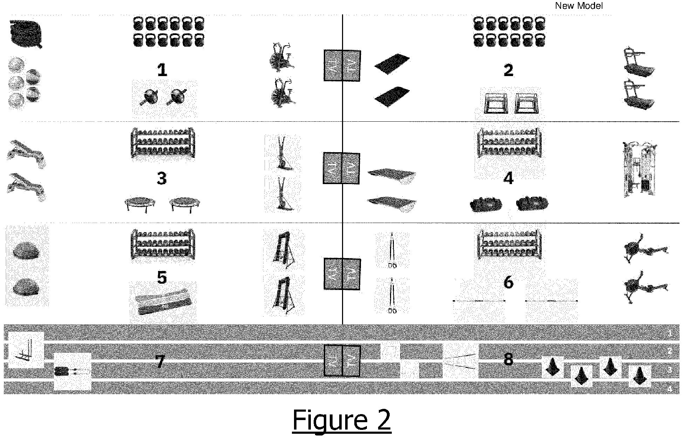

In each of the stations, equipment and accessories are distributed that, together with the screen present in this particular station, direct the exercise and stimulus for that particular day. As examples of assemblies of the 8 stations, we have figures 1 and 2 attached.

Each station comprises equipment and accessories for a type of exercise, which are selected by the technical staff according to the exercise periodization, comprising: leg exercises, chest exercises, back exercises, abdominal exercises, shoulder exercises, triceps exercises, biceps exercises and aerobic exercises. According to one embodiment of the invention, a training site with 8 exercise stations may comprise 3 or more types of exercises, preferably up to 5 types of exercises in each station.

The number of users per station may vary since for each type of exercise there may be a multiplicity of equipment and or accessories, as indicated in the attached Figure 2.

Figure 1 presents a training site with 8 stations each comprising:
- station 1 comprises three types of leg equipment and accessories, such as: smith, bars and washers, ductwork and abductor;
- station 2 comprises three types of chest equipment and accessories, such as: chest press, bars and washers, crucifix;
- station 3 comprises three types of equipment and accessories for the back, such as: seated rowing, bars and washers, lat pulldown;
- station 4 comprises three types of equipment and accessories for abdomen, such as: sitting abdomen, mattresses and washers, Bosu with ABD;
- station 5 comprises three types of shoulder equipment and accessories, such as: seated development, bars and washers, cross over (variations);
- station 6 comprises three types of equipment and accessories for triceps, such as: cross over (variations), bars and washers, triceps device on the bench;
- station 7 comprises three types of equipment and accessories for biceps, such as: Scott thread, bars and washers, cross over (variations);
- station 8 comprises three types of equipment and accessories for aerobics, such as: hammer treadmill, laddermil, paddle.

Figure 2 presents a training site with 8 stations comprising:
- in station 1: equipment and accessories for airbike, kettlebell, naval rope, Swiss ball, abdominal wheel;
- at station 2: equipment and accessories for mattress, kettlebell, box, hammer conveyor belt;
- in station 3: equipment and accessories for step, dumbbell, mini trampoline, rowing machine;
- at station 4: equipment and accessories for dumbbell, powerbag, balance platform, crossover;
- at station 5: equipment and accessories for Bosu, dumbbell, laddermill, bands;
- at station 6: equipment and accessories for TRX, dumbbell, paddle, flexibar;
- at station 7: equipment and accessories for sled, traction belt;
- at station 8: cones, ladder, barrier.

According to one embodiment of the present invention, the method for group exercise training in circuit form comprises for each user the following steps:
(a) choose through an application on the mobile or computer or tablet, the day, time, the exercise program of the day (which will indicate which will be the starting station of the exercise) and the position at the indicated starting station, provided that it is available and has not been pre-scheduled by another user;
(b) guide through a screen with an electronic personal trainer, in each of the 3 to 16 stations each of the users, where on this screen it has the identification of the user, in order to perform at least 3 types of exercises, totaling the time of 5 to 20 minutes in each station, with at least 3 sets of repetitions for each exercise per station; totaling an exercise program of 30 to 80 minutes, two different exercise programs can be performed per day, every 10 minutes starting a new exercise program, and the two different exercise programs occur simultaneously in alternating or subsequent stations, which are defined in alternating or subsequent stations according to the periodization of each exercise plan carried out by the technical staff;
(c) to wait for the time of user transitions between stations, which may vary from 10s to 90s;
(d) to start the training at the initial station that was indicated according to the exercise program chosen to perform on the day, as identified by the user on the screens;
(e) to complete the training at the last indicated station;
employing a software that:
- allows for the guidance of the exercises through an electronic personal trainer and the rotation of the exercise, being able to start in a new station a new exercise block every 5 to 20 minutes; and a new exercise program every 5 to 20 minutes;
- makes it possible for two different exercise programs to occur simultaneously, using different stations.
- controls: the interval time between one set and another, the transition time between one exercise and another, the transition time between one station and another;
- allows for the visualization on the screens of the correct way of performing the exercises, through the electronic personal trainer.

The present invention further contemplates a system for group exercise training in circuit form comprising for each user the following steps:
(a) to choose through an application on the mobile or computer or tablet, the day, time, the exercise program of the day (which will indicate which will be the starting station of the exercise) and the position at the indicated starting station, provided that it is available and has not been pre-scheduled by another user;
(b) to guide through a screen with an electronic personal trainer, in each of the 3 to 16 stations each of the users, where on this screen it has the identification of the user, in order to perform at least 3 types of exercises, totaling the time of 5 to 20 minutes in each station, with at least 3 sets of repetitions for each exercise per station; totaling an exercise program of 30 to 80 minutes, two different types of exercise program can be performed per day, every 10 minutes starting a new exercise program, and occurring simultaneously in alternating or subsequent stations, which are defined in alternating or subsequent stations according to the periodization of each exercise plan carried out by the technical staff;
(c) to wait for the time of user transitions between stations, which may vary from 10s to 90s;
(d) to start the training at the initial station that was indicated according to the exercise program chosen to perform on the day, as identified by the user on the screens;
(e) to complete the training at the last indicated station;
employing a software that:
- allows for the guidance of the exercises through an electronic personal trainer and the rotation of the exercise, being able to start in a new station a new exercise block every 5 to 20 minutes; and a new exercise program every 5 to 20 minutes;
- makes it possible for two different exercise programs to occur simultaneously, using different stations.
- controls: the interval time between one set and another, the transition time between one exercise and another, the transition time between one station and another;
- allows for the visualization on the screens of the correct way of performing the exercises, through the electronic personal trainer.

The following are examples for illustrative purposes only, and they should not be subject to limitation of the scope of the invention.

### EXAMPLES

### Example 1

The user registers in the application and chooses exercise plan A, at 7:00 am starting at station 1 and chooses their position A1 at the starting station. They arrive at the training site at least 10 minutes in advance and checks in via app or QR present on site and is ready to enter the starting station of exercise program A at position A1. The exercises may vary every day and according to the technical staff, and the modality, types of exercises and equipment used for the exercise. At 7:00 am, they start at station 1 and perform three sets of three different exercises. Each exercise lasts for 40s and a transition from one set to another of 20s. At the end of station 1 there's 60s of transition to the next station. At 7:10 am, they start at station 2, perform all exercises until 7:19 am and transition again to another station, number 3 (always observing the instructions on the screen of each station). At 7:20 am, they start their exercise at station 3. At 7:29 am, they finish the 3 sets of 3 exercises in station 3 and transitions to station 4. At station 4 they start at 7:30 am. They finish the exercises at 7:39 am, having 1 minute of rest at the end. After finishing the exercise, they have the option of going to the recovery station to recover muscles for up to 10 minutes.

### Example 2

An example of the journey of two users in the gym that comprises two different types of exercise programs, which occur simultaneously, and each exercise program uses 4 different stations of the 8 available:

User X registers in the application and chooses the time 7:00 am. User X has two exercise program options to perform on the day, exercise program A and exercise program B, of which they choose exercise program B. The app or site advisor indicates which will be the starting station of the exercise according to the chosen exercise program, the starting station will be 1 and User X chooses their position in it, position A1. On that day, the exercise stations selected for exercise program B were station 1, 4, 5, and 8. User X arrives at the training site 10 minutes in advance, checks in via app or via QR available on site, and is ready to enter station 1, where exercise program A will start, and in the position of their choice A1. The stations to be used and the type of exercise may vary every day according to the exercise periodized by the technical staff and the exercise program chosen. The exercise is based on a mix of modalities, types of exercises and equipment to be used for the exercise. At 7:00 am, User X starts at station 1, where their name is identified on the station screen, and the exercises to be done are demonstrated in it through an electronic personal trainer, which shows the correct performance of each of the exercises in each station. User X performs three sets of three different exercises. Each exercise lasts for 40s and a transition from one set to another of 20s. At the end of station 1 there's 60s of transition to the next station, which will be indicated on the monitor (or screen) as station 4, which starts at 7:10 am. User X performs all exercises until 7:19 am and transition again (for the time of up to 60 seconds) to station 5 at 7:20 am. At 7:29 am, they finish the 3 sets of 3 exercises in station 5 and transition to station 8. It starts at 7:30 am at station 8. User X finishes the exercises at 7:39 am, having 1 minute of rest at the end. After finishing the exercise, they can choose to go to the recovery station to recover their muscles and being able to stay up to 10 minutes. Simultaneously, user Y registers in the application and chooses the time 7:00 am. User Y has two exercise program options to perform on the day, exercise program A and exercise program B, they choose exercise program A. The app or site advisor indicates which will be the starting station of the exercise according to the chosen exercise program, the starting station will be 2 and User Y chooses their position in it, position B2. On that day, the exercise stations selected for exercise program B were station 2, 3, 6, and 7. User Y arrives at the training site 10 minutes in advance, checks in via app or via QR available on site, and is ready to enter station 2, where exercise program B will start, and in the position of their choice A1. The stations to be used and the type of exercise may vary every day according to the exercise periodized by the technical staff and the exercise program chosen. The exercise is based on a mix of modalities, types of exercises and equipment to be used for the exercise. At 7:00 am, User Y starts at station 2, where their name is identified on the station screen, and the exercises to be done are demonstrated in it through an electronic personal trainer, which shows the correct performance of each of the exercises in each station. User Y performs three sets of three different exercises. Each exercise lasts for 40s and there's a transition from one set to another of 20s. At the end of station 2 there's 60s of transition to the next station, which will be indicated on the screen as station 3, in which they start at 7:10 am. User Y performs all exercises until 7:19 am and transitions again (for the time of up to 60 seconds) to station 6 at 7:20 am. At 7:29 am, they finish the 3 sets of 3 exercises in station 6 and transitions to station 7. It starts at 7:30 am at station 7. They finish the exercises at 7:39 am, having 1 minute of rest at the end. After finishing the exercise, the user can choose to go to the recovery station to recover their muscles being able to and stay up to 10 minutes.

## Claims

1. A method for improving circuit physical training, **characterized by** comprising for each user the following steps:
(a) choosing through a mobile or computer or tablet application, the day, time, the exercise program of the day (which will indicate which will be the starting station of the exercise) and the position at the indicated starting station, provided that it is available and has not been pre-scheduled by another user;
(b) guiding through a screen with an electronic personal trainer, in each of the 3 to 16 stations each of the users, where on this screen it has the identification of the user, in order to perform at least 3 types of exercises, totaling the time of 5 to 20 min in each station, with at least 3 sets of repetitions for each exercise per station; totaling an exercise program of 30 to 80 minutes, two different exercise programs being available to be performed per day, starting a new exercise program at each interval of 5 to 20 min, and occurring simultaneously in alternating or subsequent stations according to the periodization of each exercise plan carried out by the technical staff;
(c) waiting for the time of user transitions between stations, which may vary from 10s to 90s;
(d) starting the training at the initial station, which was indicated according to the exercise program chosen to perform on the day, as identified by the user on the screens;
(e) completing the training at the last station indicated.

2. The method for improving circuit physical training according to claim 1, **characterized by** the transition time from exercise to exercise within a same station being able to range from 10s to 60s, and the transition time from one station to another station being able to range from 10s to 90s.

3. The method for improving circuit physical training according to claim 1, **characterized by** employing a software that:
- allows for the guidance of the exercises through an electronic personal trainer and the rotation of the exercise, being able to start in a new station a new exercise block every 5 to 20 minutes; and a new exercise program every 5 to 20 minutes;
- allows for the occurrence of two different exercise programs occurring simultaneously, using different stations;
- controls: the interval time between one set and another, the transition time between one exercise and another, the transition time between one station and another;
- allows for the visualization on the screens of the correct way of performing the exercises, through the electronic personal trainer.

4. The method for improving circuit physical training according to claim 1, **characterized in that** the user can start a new exercise program every 10 minutes.

5. The method for improving circuit physical training according to claim 1, **characterized by** exercises that may or may not be divided into exercise types by muscle groups on weekdays, or according to the training periodization established by the technical staff.

6. The method for improving circuit physical training according to claim 1, **characterized by** employing a mix of stimuli from strength training, endurance, strength endurance, flexibility, bodybuilding, cardiovascular, aerobic, anaerobic, fitness, physical therapy, balance, mobility, functional, HIIT, calisthenics, stretching, among others.

7. The method for improving circuit physical training according to claim 1, **characterized in that** each station comprises equipment and accessories for one type of exercise, which were selected by the technical staff according to the periodization of the exercise, comprising: exercises for the legs, exercises for the chest, exercises for the back, exercises for the abdomen, exercises for the shoulder, exercises for the triceps, exercises for the biceps and aerobic exercises.

8. The method for improving circuit physical training according to claim 1, **characterized in that** the training site comprises stations with exercises chosen from the group comprising: weight training, aerobics, wrestling, functional, cross training, cardio, Pilates, yoga, ginástica natural or natural gymnastics, dance, muay Thai, boxing, jiu-jitsu, spinning, running, air bike, flexibility, HIIT. Material and exercise Dumbbell, dumbbell, airbike, step, jump, treadmill, naval rope, spinning bike, exercise bike, ball with weight, TRX, mat, kettlebell, elastic, paddle, balance platform, Bosu, sled, fighting bob, rope, tire, small round cone, small triangular cone, balance board, exercise ladder, plyometric box, ladder, ladder mill, among others.

9. The method for improving circuit physical training according to claim 1, **characterized by** comprising an exercise program of up to 40 minutes distributed over 8 exercise stations.

10. The method for improving circuit physical training, according to claim 1, **characterized in that** the training is carried out in a place that comprises two exercise programs in a total of 8 exercise stations, preferably 4 stations per exercise program are used simultaneously, according to the periodization of the exercises of that given day, determined by the technical staff.

11. Method for improving circuit physical training, according to claims 1 or 10, **characterized in that** the training site comprises 8 stations, with 8 stations being used simultaneously, with two different exercise programs that use 4 stations for each program, comprising two different types of 40-minute exercise program, which occur simultaneously, and each exercise program uses 4 exercise stations, totaling 8 stations available, where 3 sets of 40 seconds of each exercise are performed with a transition period for 20-second rest in each station.

12. The method for improving circuit physical training, according to claim 1, **characterized in that** the training site presents the distributed stations, side by side, of free transit for the users, there are no walls or structures that isolate each station leaving free the transit of the users between the stations, or being able to present the 4 stations on the ground floor of free transit for the users, and 4 stations of free transit on the mezzanine or first floor.

13. The method for improving circuit physical training according to claim 1, **characterized by** comprising for each user the following steps:
(a) choosing through a mobile or computer or tablet application, the day, time, the exercise program of the day (which will indicate which will be the starting station of the exercise) and the position at the indicated starting station, provided that it is available and has not been pre-scheduled by another user;
(b) guiding through a screen with an electronic personal trainer, in each of the 3 to 16 stations each of the users, where on this screen it has the identification of the user, in order to perform at least 3 types of exercises, totaling the time of 5 to 20 minutes in each station, with at least 3 sets of repetitions for each exercise per station; totaling an exercise program of 30 to 80 minutes, two different exercise programs being available to be performed per day, every 10 minutes starting a new exercise program, and occurring simultaneously in alternating or subsequent stations, which are defined in alternating or subsequent stations, according to the periodization of each exercise plan carried out by the technical staff;
(c) waiting for the time of user transitions between stations, which may vary from 10s to 90s;
(d) starting the training at the initial station that was indicated according to the exercise program chosen to perform on the day, as identified by the user on the screens;
(e) completing the training at the last indicated station;
employing a software that:
- allows for the guidance of the exercises through an electronic personal trainer and the rotation of the exercise, being able to start in a new station a new exercise block every 5 to 20 minutes; and a new exercise program every 5 to 20 minutes;
- allows for the occurrence of two different exercise programs occurring simultaneously, using different stations;
- controls: the interval time between one set and another, the transition time between one exercise and another, the transition time between one station and another;
- allows for the visualization on the screens of the correct way of performing the exercises, through the electronic personal trainer.

14. A system for improving a circuit physical training method according to claim 1, **characterized by** comprising for each user the following steps:
(a) choosing through a mobile or computer or tablet application, the day, time, the exercise program of the day (which will indicate which will be the starting station of the exercise) and the position at the indicated starting station, provided that it is available and has not been pre-scheduled by another user;
(b) guiding through a screen with an electronic personal trainer, in each of the 3 to 16 stations each of the users, where on this screen it has the identification of the user, in order to perform at least 3 types of exercises, totaling the time of 5 to 20 minutes in each station, with at least 3 sets of repetitions for each exercise per station; totaling an exercise program of 30 to 80 minutes, two different exercise programs being available to be performed per day, every 5 to 20 minutes starting a new exercise program, and occurring simultaneously in alternating or subsequent stations, which are defined in alternating or subsequent stations, according to the periodization of each exercise plan carried out by the technical staff;
(c) waiting for the time of user transitions between stations, which may vary from 10s to 90s;
(d) starting the training at the initial station that was indicated according to the exercise program chosen to perform on the day, as identified by the user on the screens;
(e) completing the training at the last indicated station;
employing a software that:
- allows for the guidance of the exercises through an electronic personal trainer and the rotation of the exercise, being able to start in a new station a new exercise block every 5 to 20 minutes; and a new exercise program every 5 to 20 minutes;
- allows for the occurrence of two different exercise programs occurring simultaneously, using different stations;
- controls: the interval time between one set and another, the transition time between one exercise and another, the transition time between one station and another;
- allows for the visualization on the screens of the correct way of performing the exercises, through the electronic personal trainer.
